# EUROPEAN PATENT SPECIFICATION

(11) **EP 2 093 280 B1**
(45) Date of publication and mention of the grant of the patent: **11.05.2011**
(21) Application number: 08003412.7
(22) Date of filing: 25.02.2008
(51) Int. Cl.: C12N 9/00, C12N 9/48, C12N 15/85, A01K 67/027

(54) **Non-human model animal lacking mast cells**
Nicht-menschliches Modelltier mit selektivem Fehlen von Mastzellen
Animal modèle non-humain manquant de manière sélective de mastocytes

(43) Date of publication of application: 26.08.2009
(73) Proprietor: Rodewald, Hans-Reimer, Prof. Dr., 89075 Ulm (DE)
(72) Inventor: Rodewald, Hans-Reimer, Prof.Dr., D-89075 Ulm (DE); Feyerabend, Thorsten, Dr., D-89075 Ulm (DE)
(74) Representative: Pfenning, Meinig & Partner GbR

(56) References cited:
- MCCORMACK MATTHEW P ET AL: "The LMO2 T-cell oncogene is activated via chromosomal translocations or retroviral insertion during gene therapy but has no mandatory role in normal T-cell development." MOLECULAR AND CELLULAR BIOLOGY, vol. 23, no. 24, December 2003 (2003-12), pages 9003-9013, XP002489981 ISSN: 0270-7306
- SCHOLTEN JULIA ET AL: "Mast cell-specific Cre/loxP-mediated recombination in vivo" TRANSGENIC RESEARCH, vol. 17, no. 2, 31 October 2007 (2007-10-31), pages 307-315, XP002489982 ISSN: 0962-8819
- THORSTEN FEYERABEND,ET AL.: "Deletion of NOTCH-1 in KIT+ PRO-T cells blocks T Cell Development, but does not convert T cell progenitors into thymic B cells" 37TH ANNUAL MEETING OF THE GERMAN SOCIETY FOR IMMUNOLOGY, [Online] 5 September 2007 (2007-09-05), - 8 September 2007 (2007-09-08) page 219, XP002489985 Heidelberg, Germany Retrieved from the Internet: URL:http://www.dgfi.dbkr.de/fileadmin/pdf/ dgfi_abstracts_fall07.pdf> [retrieved on 2008-07-24]
- SCHMIDT-SUPPRIAN MARC ET AL: "Vagaries of conditional gene targeting." NATURE IMMUNOLOGY JUL 2007, vol. 8, no. 7, July 2007 (2007-07), pages 665-668, XP002489983 ISSN: 1529-2908
- DEPINAY N ET AL: "Mast cell-dependent down-regulation of antigen-specific immune responses by mosquito bites" JOURNAL OF IMMUNOLOGY 20060401 US, vol. 176, no. 7, 1 April 2006 (2006-04-01), pages 4141-4146, XP002489984 ISSN: 0022-1767

## Description

The present invention is related to a non-human model animal showing a lack of mast cells, whose genome comprises, inserted into an endogenous Mc-cPA allele ("knocked-in"), a Cre-recombinase expression cassette. The invention also relates to a method for producing this animal and use thereof.

Mice selectively lacking particular cell types or organs are of great value for basic research and for the biomedical and pharmaceutical industry. Famous examples are the 'nude' mouse that has no thymus, or recombinase gene-deficient mice both of which are immunodeficient and can e.g. be used as graft recipients.

One example of a particular cell type being of great interest in the basic research are mast cells. Mast cells originate from hematopoietic stem cells, and therefore belong to the group of blood and immune cells. Mast cells are famous for their detrimental contributions to general health problems such as allergy and asthma. Moreover, mast cells are thought to be broadly involved in innate and adaptive immune responses. As such, mast cells have been speculated to protect from, or to exacerbate many pathological processes including bacterial infections and sepsis, tumor immunology, autoimmunity in models of arthritis and multiple sclerosis, tolerance in a skin graft rejection model, vascular diseases, or wound healing.

Essentially all data on the above proposed in vivo functions of mast cells were derived from experiments using the available mast cell-deficient mouse models. These models depend on mutations in the receptor tyrosine kinase Kit that is a crucial growth factor receptor for mast cells. Therefore, all currently available mast cell-deficiency models rely on mutant Kit alleles that impair various functions of this receptor. However, the Kit receptor is not only expressed in mast cells but also in many other cell types including germ cells, hematopoietic stem and progenitors cells, melanocytes, neuronal cells, intestinal pacemaker cells (interstitial cells of Cajal), and postnatal liver cells. According to this widespread expression pattern, and in keeping with the important and pleiotropic roles of Kit in the development or function of these cell types, mouse mutants in which Kit expression, or its signaling are perturbed show multiple and severe defects. These include impaired fertility (germ cells), reduced blood cell production causing anemia (hematopoietic stem and progenitors cells), white fur color (melanocytes), sensory deficits (neuronal cells), impaired gut mobility (intestinal pacemaker cells), and defects in lipid metabolism (liver cells).
The most frequently used mast cell-deficient mouse, the WBB6-Kit^{W/Wv} mutant e.g. Depinay N, et al (2006), The Journal of Imunology, vol. 176, pp. 4141-4146, suffers phenotypically from many Kit defects including the severe anemia, absence of melanocytes in the skin, and the gut motility defect. Of note, while mast cells can at least partially be reconstituted in WBB6-Kit^{W/Wv}mice by injection of normal mast cells, additional Kit-dependent defects cannot be overcome in WBB6-Kit^{W/Wv} mice. Moreover, WBB6-Kit^{W/Wv} mice must be generated as F1 animals from two parental strains, the WB strain and the C57BL/6 (B6) strain. The genetic background of mice is very important for immunological studies, and many other physiological and pathological studies in general, and WBB6 F1 animals are only poorly defined. Therefore, it would be important that mast cell-deficient mice are available on different genetic backgrounds including strains that are inherently prone for certain diseases. Crossing the common mast cell-deficiency mutations (Kit^{W/Wv}) on various pure genetic backgrounds is very cumbersome because the Kit-mutations are recessive, e.g. mast cell-deficiency requires both alleles. Since homozygous animals are infertile (Kit^{Wv}) or not viable (Kit^{W}), further crosses to transgenic or knockout mice are very complex, and in fact not practical. This is evident from the fact that WBB6-Kit^{W/Wv} mice have been used for about 30 years, and there are very few, if any, studies that have transferred the Kit-dependent mast cell-deficiency to other genetic backgrounds.

A second, and more recently introduced mast cell-deficiency model is based on a different Kit allele, called Kit^{W-Sh} ("W shash"). These mice have several advantages over WBB6-Kit^{W/Wv} mice because they do not have the full list of Kit mutant phenotypes. Kit^{W-Sh} mice are thought to be on the B6 background, however, there is some histo-incompatibility between Kit^{W-Sh} and B6 as shown by rejection of B6 donor skin in Kit^{W-Sh} recipient mice. Kit^{W-Sh} mice are certainly more convenient because they can be bred, and have no anemia. This strain, however, still suffers from the gut motility defect, lacks skin melanocytes, and may have other defects in Kit-driven cells or organs that have not been tested yet (neurons, pain sensitization, liver metabolism and others). Moreover, Kit^{W-Sh} mice do have mast cells at young age, and only loose these during adult life.
Interestingly, as investigators are beginning to compare these two models (WBBG-Kit^{W/Wv} versus B6-Kit^{W-Sh} mice), significant differences emerge. In a model of antibody-driven arthritis, WBB6-Kit^{W/Wv} mice were resistant while B6-Kit^{W-Sh} mice were susceptible. This strongly suggests that factors other than the presence or absence of mast cells may have important impact on the outcome in studies on the physiological or pathological functions of mast cells. In this case, the results question the crucial role of mast cells in this model of joint pathology, or, at least, underscore the 'model-dependency' of such conclusions. The quoted very recent example exemplifies that a better-defined mouse lacking only mast cells will be required to gain definitive information on mast cell functions in vivo.
Finally, it is important to note that the absence of mast cells may not be absolute as mast cell populations can be induced under certain inflammatory conditions within a few days in WBB6-Kit^{W/Wv}, and possibly also in B6-Kit^{W-Sh} mice.

The document Scholten J et al (2007), Transgenic Research, vol. 17, pp. 307-315 describes transgenic mice expressing Cre recombinase under the control of the mast cell protease (Mcpt) 5 promoter.
The document Feyerabend T et al (2007), 37th Annual Meeting of the German Society for Immunology (http://www.dgfi.dbkr.de/fileadmin/pdf/dgfi_abstracts_fall07.pdf), page 219, describes a Cre recombinase mouse (Mc-cpa^{Cre}) in which Cre expression is controlled by a myeloid gene locus (mast cell-carboxypeptidase A [Mc-cpa]). No direct and unambigous mention is made of insertion of the Cre-recombinase coding sequence into an endogenous Mc-cpa allele, or of absence of mast cells.

The object of the present invention is therefore to provide a further non-human model animal, which lacks mast cells Further, this non-human model animal should be easily bred to generate mast cell deficiency in several pure mouse genetic backgrounds, and to be able to combine the specific cell type deficiency with other mutations. The mast cell deficient non-human animal model (Cre-Master mouse) according to the invention lacks all mast cell populations such as connective tissue mast cells in the skin and the peritoneal cavity as well as mucosal mast cells in the gut. Further, the mast cell deficiency should not be overcome by acute or chronic inflammation or other pathological conditions. This object is solved by the non-human animal model, the method for producing such an animal as well as use of such a model for testing according to the present independent claims. Preferred embodiments are given in the dependent claims. As described in detail below, by means of the Cre-Master mouse of the invention, this object is solved and the present invention provides a novel tool to study mast cell type functions.

Up to now it has been suggested that Cre toxicity can cause DNA damage by attack of Cre on "endogenous loxP-like" sites. Cre-mediated DNA breaks should lead to cell cycle arrest and cell death by apoptosis. This property of Cre has been viewed as problematic, and therefore negative, rather than advantageous, and it has not yet been used as a tool to abrogate specific cell lineages in vivo.

The inventors of the present invention provide now a new method for generation of a non-human model animal which lacks mast cells, as recited in the claims A prerequisite for Cre-mediated mast cell ablation as demonstrated in this invention is that Cre is inserted into an endogenous MC-CPA allele ('Knock-in') driving mast cell-type-specific expression of Cre, and that Cre expression from such allele is very strong and long lasting.

For the first time the inventors of the present invention have generated a mouse mutant bearing the enzyme Cre-recombinase (Cre) gene in an endogenous locus that is specifically expressed in peripheral tissues in mast cells (mast cell-protease locus) and the expression of which eliminates exclusively mast cells. The chosen locus is the mast cell carboxypeptidase A (Mc-cpa) gene. Cre originates from bacteriophages, and is known for its capacity to re-combine genomes at target sequences called 1oxP sites. Usually, Cre is inserted in transgenic mice in which Cre expression is used to delete DNA segments between 1oxP sites that need to be introduced separately into the mouse genome. This implies that Cre normally acts with high specificity on single loxP-flanked gene loci in the entire genome.
Cre has been inserted by gene targeting via homologous recombination in embryonic stem (ES) cells into the Mc-cpa locus. After germline transmission of this new Mc-cpa allele (Mc-cpa^{Cre}), mice developed that had no mast cells. The effect of Cre in these mice is independent of any artificial 1oxP sites because 1oxP sites have not been introduced into these animals. Mc-cpa is expressed at very high levels in mast cells, and surprisingly it turned out that in Mc-cpa^{Cre} mice, strong or long-lasting expression of Cre fully eliminates mast cells by destroying their DNA. Collectively, dose and duration of Cre expression from this locus are genotoxic for mast cells, and cause a complete absence of mast cells in this mouse strain. This new mast cell-deficient mouse strain is termed 'Cre-Master_{'} mice for 'Cre-mediated mast cell eradication'.

### Detailed Description

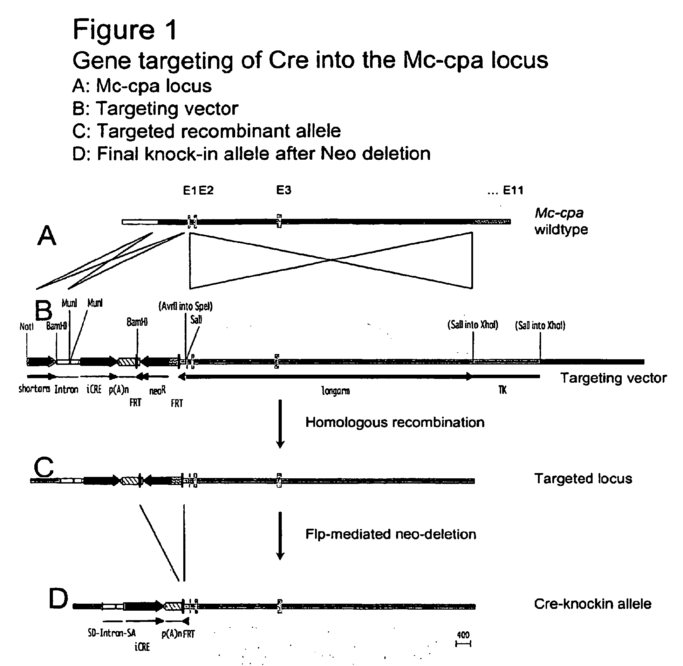

The *Mc-cpa* gene locus encodes Mc-cpa, a zinc-metallo protease that is strongly expressed by mast cell progenitors, and in mature mast cells. In addition to mc-cpa^{Cre} mice, also a 'reporter mouse' has been generated in which transcription from the Mc-cpa locus can be visualized by cell surface expression of the marker gene-derived protein, and by which it can be demonstrated that Mc-cpa is both very strongly and highly specifically expressed in mast cells.
The Mc-cpa protein is stored inside the mast cells' secretory granules. As shown previously the deletion of the Mc-cpa gene in knockout mice has no major effect on mast cell functions or numbers. The *Mc-cpa* gene (Figure 1A) consists of eleven exons, spanning a distance of 25.56 kb. The transcript has a short 5' untranslated region (10 nucleotides) and a total length of 1452 bps, coding for a 417 amino acid long pre-pro enzyme (precursor enzyme). After cleavage of the 15 amino acid long N-terminal signal peptide and a 94 amino acid long activation peptide, the core enzyme has a molecular weight of 36 kDa.

The targeting construct is outlined in Figure 1B. It is based on the plasmid pBluescript SK(-)(Stratagene) and consists, from 5' to 3', of the following components:
A "short arm", an intronic sequence from rabbit β-globin, the coding sequence of the Cre-recombinase, the poly-adenylation signal from rabbit β-globin, the frt-flanked neomycine resistance gene, the "long arm" and the thymidine kinase gene. Instead of the neomycine resistance gene other resistance genes can be used such as e.g. puromycin, hygromycin, bleomycin, hprt or dhfr. Instead of the thymidin kinase gen e.g. diphtheria toxin can be used.
The short arm and the long arm are sequences homologous to the nucleotides -808 to +11 and +40 to +8034 (according to ensemble mouse genome database) of the *Mc-cpa* gene, respectively and are required for site-specific integration of Cre-recombinase into the Mc-cpa locus. They were amplified by PCR from a 129/SvJ bacterial artificial chromosome (BAC#24998, Incyte Genomics). The PCR primers were designed according to the published *Mc-cpa* sequence (AC068496, Mus musculus strain C57BL/6J chromosome 3 clone RP23-75F13 complete sequence) and contained the appropriate restriction-sites for subsequent cloning of the PCR products. The intronic cassette in front of the Cre-recombinase open-reading frame (ORF) was used to improve mRNA stability. It contains an intronic sequence of rabbit β-globin together with the corresponding splice-donor and splice-acceptor sequences. The codon-improved Cre-recombinase (iCre) the ORF of which spans 1055 nucleotides (Shimshek et al., 2002, Genesis 32:19-26) was used. The cassette with the frt-flanked neomycin resitance gene for positive selection of transfected embryonic stem cells was taken from pFRT-neo-FRT (Luche, 2007, Dissertation, University Ulm). Frt-sites (gaagttcctattctctagaaagtatag-gaacttc, palindromic sequence underlined) were included to enable removal of the neomycin resistance cassette by transient Flp-recombinase expression in the targeted ES cells. The thymidine kinase gene was cloned from pCAU-8.

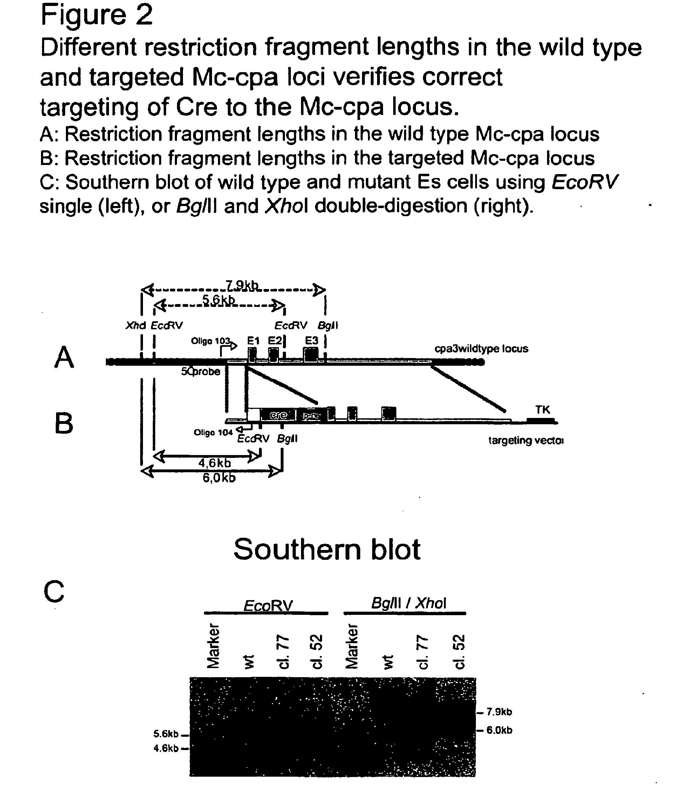

After transfection of the targeting vector (Figure 1B) into ES cells, the DNA sequence in between the two homologous arms of the vector was integrated site-specifically into the first exon of the endogenous *Mc-cpa* gene (Figure 1A) via homologous recombination (Figure 1C). Correctly targeted ES cell clones were identified by a diagnostic polymerase chain reaction (PCR) (not shown). The restriction fragments indicative of the wild type (Figure 2A), and the correctly targeted (Figure 2B) loci were demonstrated by southern blotting (Figure 2C). This insertion results in expression of Cre precisely under the control of the *Mc-cpa* promoter. The only remaining further genetic alteration is a concomitant deletion of 28 nucleotides of the first exon of *Mc-cpa.*

By transient transfection and expression of the Flp-recombinase, the neomycin resistance cassette was removed from the targeted locus. A single FRT-site remains in the genome (Figure 1D). ES cells bearing this final Cre-knock-in allele were injected into C57BL/6 blastocysts. Chimeric mice backcrossed to C57BL/6 mice transmitted the Mc-cpa^{Cre} allele through the germline. All agouti animals in a litter (F1) are ES cell-derived and were screened for the targeted allele. Heterozygous mice represent the founder mice of the newly generated Cre-Master mouse. The Mc-cpa^{Cre} allele was backcrossed over 12 generations onto two very commonly used mouse backgrounds, the C57BL/6 and the BALB/c strains. At this time point (12/2007), essentially pure C57BL/6 and BALB/c strains lacking mast cells were obtained.

### Preparation of Cre-Master mice

### Generation of the targeting construct:

Generation of the targeting construct required several cloning steps. First, two separate plasmid vectors were cloned: One containing a thymidine kinase gene for negative selection and the long arm of homology (pBSK-AD), the second vector containing the short arm, the Cre-recombinase expression cassette and the Frt-flanked neomycin resistance gene (pBSK-BE). Finally, the targeting construct was obtained by inserting the said elements of the second vector into the first.

Molecular biology procedures were the standard techniques described in the laboratory manual of Sambrook and Maniatis (Sambrook, J., E. F. Fritsch, and T. Maniatis, 1989, Molecular Cloning: A Laboratory Manual, Cold Spring Harbor, New York, Cold Spring Harbor Laboratory).

The homologous short- and long arm were amplified by PCR from a 129/SvJ bacterial artificial chromosome (BAC#24998, Incyte Genomics). The PCR primers were designed according to the published Mc-cpa sequence (AC068496, Mus musculus strain C57BL/6J chromosome 3 clone RP23-75F13 complete sequence). At the 5' ends of the primers, specific restriction sites were included for subsequent cloning of the PCR products.

### List of Oligos:

| | |
|---|---|
| Oligo 50: | TATGGCTGTCGACTACACTACACTTGCAATTGCTCC |
| Oligo 82: | TATGGCTGTCGACTAGGATGAGCCTGAACGCAGG |
| Oligo 93: | TATTAGCGGCCGCGAAATCTGAGTTCGAGACCAGC |
| Oligo 103: | CTTTAATTCCAGCACTTGGATTTCG |
| Oligo 104: | CCGGACACGCTGAACTTGTGGC |
| Oligo 127: | GCAATAGCTTGTCCACCCAAC |
| Oligo 137: | GAAATCTACTGTCCTTTGCCTC |
| Oligo 316: | CCTAGGATCCTGTTTTTCTTGGCTTGATCTGACCCC TATAC |
| Oligo 327: | GATCCCGGGCTCGAGGCGCGCCTAGGTAC |
| Oligo 328: | CTAGGCGCGCCTCGAGCCCGG |
| Oligo 329: | AATTGTCCGGACACGCTGAACTTGTGGC |
| Oligo 330: | AATTGCCACAAGTTCAGCGTGTCCGGAC |
| Oligo 342: | TCGACACTAGTACCGGTGC |
| Oligo 343: | GGCCGCACCGGTACTAGTG |

### Three step cloning of Vector#1 (pBSK-AD)

In the first step, oligos 342 and 343 were hybridized and inserted as a polylinker into the NotI and SalI digested plasmid pBlueskript SK(-). Subsequently, the resulting plasmid was digested with XhoI and the thymidine kinase gene was introduced as a 1.8 kb Sa-1I+XhoI fragment from pCAU-8. Finally, the resulting plasmid was digested with SalI and XhoI and the 8 kb long arm, which had been amplified by PCR using oligos 50 and 82 and subsequently been digested with SalI, was inserted to obtain vector#1 (pBSK-AD).

### Five step cloning of Vector#2 (pBSK-BE):

First, the hybridized oligos 327 and 328 were cloned as a polylinker into the BamHI and KpnI digested plasmid pBlueskript SK(-). In the next step, the resulting plasmid was digested with NotI and BamHI and the short arm PCR product, amplified using oligos 93 and 316, that had been digested with the same restriction enzymes was inserted. Subsequently, the Frt-flanked neomycin resistance gene was cloned as a 1.3kb BamHI plus AvrII fragment from the plasmid pHL-IE (Luche, 2007, Dissertation, University Ulm) into the BamHI and AvrII sites. In the fourth step, this resulting plasmid was digested with BamHI and the Cre-recombinase expression cassette was inserted. The Cre-recombinase expression cassette contains a splice donor, an intronic sequence and a splice acceptor from rabbit β-globine, the codon-improved Cre-recombinase coding sequence and the rabbit β-globine polyadenylation signal. This whole cassette was excised from the plasmid pHL-IE (Luche, 2007, Dissertation, University Ulm) with BamHI. Finally, the hybridized oligos 329 and 330 were cloned into the MunI site to introduce the sequence of the oligo 104, resulting in vector #2 (pBSK-BE).

### Cloning of the targeting construct by combining vector#1 and #2:

The targeting construct was obtained by insertion of a 4.4 kb NotI plus AvrII fragment form vector#2 (pBSK-BE) into the NotI plus SpeI digested vector#1 (pBSK-AD). This final targeting vector consists from 5' to 3' of a short arm, the Cre-recombinase expression cassette, a neomycin resistance gene flanked by frt-sites, the long arm and a thymidine kinase gene. The sequence of the short arm and long arm is homologous to the nucleotides -808 to +11 and +40 to +8034 (according to ensemble mouse genome database) of the *Mc-cpa* gene, respectively.

### Generation of a recombinant Mc-cpa allele by targeted gene-knockin in ES cells in order to express the Cre-recombinase in mast cells:

### Transfection of murine ES cells and selection of clones that had undertone homologous recombination

Murine E14.1 (129/Ola) embryonic stem cells (ES cells) were cultured on neomycin resistant murine embryonic fibroblasts (MEFs) in DMEM (with Glutamax and high glucose, Gibco) supplemented with 15% FCS (Hyclone), 1 mM Pyruvate (Gibco), 100 U/ml Penicillin (Gibco), 100 µg/ml Streptomycin (Gibco), 20 µl/l MTG (Sigma) and LIF (10⁶ U/l ESGRO, Chemicon). Prior to seeding of the ES cells, MEFs forming a confluent monolayer were mitotically inactivated by γ-irradiation (3300 cGy).

ES cells were transfected by electroporation with 40 µg of the NotI-linearized targeting vector in 800 µl PBS using a Bio-Rad Gene pulser (4-mm-gap cuvette, 230 V, 500 µF). Transfected cells were selected with G418 (200 µg/ml, Gibco) and 2 µM Gancyclovir (Cymeven, Roche). Among the surviving colonies, clones that had undergone homologous recombination were identified by PCR and confirmed by southern blot. Oligos used for the PCR screen anneal to a genomic Mc-cpa sequence upstream of the short arm (Oligo #103) and to a sequence inside the targeting construct (Oligo #104). The amplified fragment (1.2 kb) thus indicates site-specific integration. Clones that were positive by PCR for site-specific integration were further analyzed for correct homologous recombination by southern blot. Sizes of the expected fragments are depicted in Fig. 2A, B. Genomic DNA (10 µg) of the ES cells was digested with XhoI+BglII or with EcoRV, separated by agarose gel electrophoresis, blotted to nylon membrane and hybridized with a *Mc-cpa* probe located outside of the targeting construct (see Fig. 2A). The probe (1.17 kb) was amplified by PCR (oligos 127 and 137) from wildtype genomic ES cell DNA and labeled with α-³²P-dCTP using the Prime-It Random Primer Labeling Kit (Stratagene). Correct integration of the targeting construct in Clones #52 and #77 was confirmed by the presence of a 4.6 kb EcoRV and a 6 kb XhoI+BglII 'knockin' fragment in addition to the respective 5.6 and 7.9 kb bands of the wildtype allele (Fig. 2C).

### In vitro deletion of the neomycin resistance gene

The neomycin resistance gene included in the targeting vector was flanked by Frt-sites, which allowed for its removal by transient expression of the Flp-recombinase. This enzyme excises DNA between two parallel Frt-sites and a single Frt-site remains in the locus. We transfected ES cells of the clone #77 with 20 µg of the plasmid pCAGGS-FLPeV1. This bicistronic plasmid allows simultaneous expression of the green fluorescent protein Egfp and the Flp-recombinase (FLPe) in eukaryotic cells. (pCAGGS-FLPeV1 was obtained from pCAGGS-FLPe by exchange of IRES-puro α-gainst IRES-Egfp). Transfection was done by electroporation, essentially as described above. Henceforth, transfected ES cells were maintained without G418. After 24-36 h, positively transfected cells were detected and enriched by FACS-sorting for Egfp expression. After culture for about 8 days, individual ES cell colonies were picked and these subclones were grown in duplicates on 96 well plates, either in the presence or absence of G418. G418-sensitive clones had deleted the neomycin resistance gene and the corresponding clones from the non-selective plates were further propagated. Neo-deletion was confirmed by PCR.

### Generation of transgenic mice containing the recombinant Mc-cpa allele

About 12-15 ES cells containing the recombinant Mc-cpa:Cre knockin allele were injected per blastocyst (C57BL/6, 3.5 dpc) and 12-16 chimeric blastocysts were transferred bilateral intra-uterine into 2.5 dpc CD1 pseudo-pregnant foster mice. Offspring with high coat color chimerism were backcrossed to C57BL/6 mice to transmit the recombinant Mc-cpa allele through the germline and thereby generate animals carrying the recombinant allele uniformly in every cell. All agouti animals in a litter (F1) are ES cell-derived and were screened for the targeted allele. These heterozygous mice represent the founder mice of the newly generated Cre-Master mouse strain. Finally, the knockin allele was backcrossed over several generations onto both, the C57BL/6 and the BALB/c backgrounds.

### Characterization of the mast cell-deficiency phenotype of Cre-Master mice

### a. Normal hematological parameters in Cre-Master mice

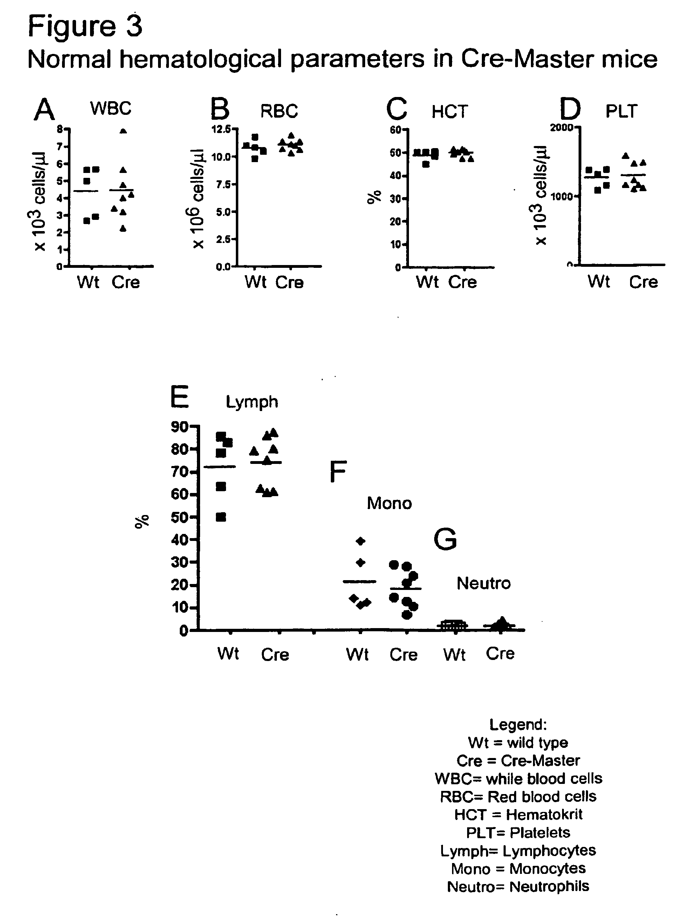

To determine a possible influence of the Creinsertion on blood cell lineages other than mast cells, detailed hematological parameters were determined in wild type and in Cre-Master mice (Figure 3). Peripheral blood analysis was performed using an automated hemocytometer according to the manufacturer's instructions (ADVIA120; Bayer, Leverkusen, Germany). One-hundred microliter EDTA-anticoagulated blood collected from the tail vein were diluted with 100µl PBS. Sample measurement was done according to the manufacturer's instructions. Specifically, numbers of leukocytes (white blood cells [WBC]) (Figure 3A), erythrocytes (red blood cells [RBC]) (Figure 3B), the hematocrit (HCT) (Figure 3C), and numbers of platelets (PLT) (Figure 3D) were measured, and the WBC were further differentiated into lymphocytes (Figure 3E), monocytes (Figure 3F), and neutrophilic granulocytes (Figure 3G). None of these parameters differed between wild type mice and Cre-Master mice demonstrating no adverse effect of the Cre insertion on the development of these major blood and immune cell types.

### b. Absence of mast cells in Cre-Master mice

### Absence of peritoneal mast cells observed by FACS-analysis

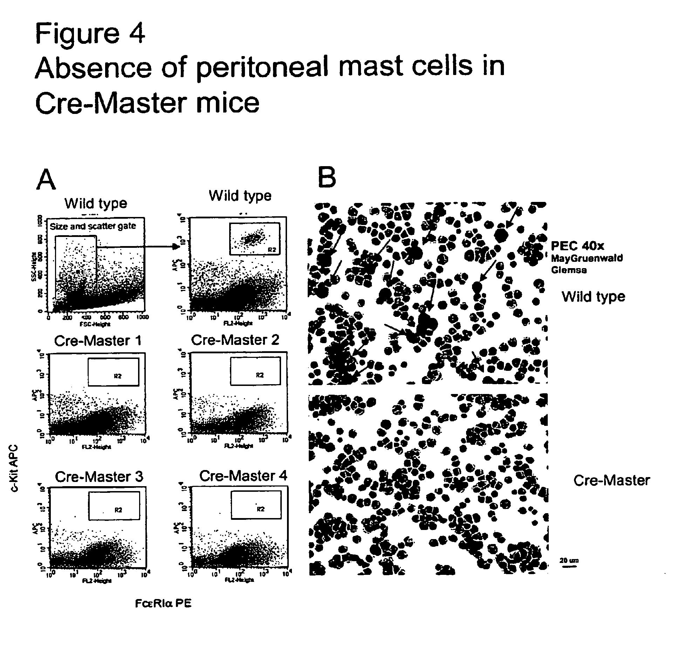

Peritoneal exudate cells (PEC) were obtained from peritoneal lavages with 10 ml pre-warmed PBS containing 5 % FCS. Subsequent incubations were performed on ice with light protection. Prior to the surface marker staining, Fcy-receptors were blocked by incubating the cells with 0.5 mg/ml mouse IgG (Dianova). Up to 5x10⁶ cells were stained with APC-labeled anti-kit (2B8, Pharmingen) and PE-labeled anti-FcεRI (MAR-1, eBiosciences) antibodies diluted in 50 µl PBS/FCS for 45 min. Subsequently, cells were washed once in 1 ml PBS/FCS. Stained cells were analyzed on a FACSCalibur instrument (Becton Dickinson); data are displayed as dot plots (Fig. 4A) using CellQuest software (Becton Dickinson).

### Absence of peritoneal mast cells observed by histology on cytospin preparations

For cytospin preparations, PEC were collected as described above and 2x10⁵ cells in 200 µl PBS/FCS were centrifuged onto glass slides (700 rpm, 5 min, low acceleration, Cytospin3, Shandon). Histochemical staining of the specimens was according to Pappenheim (Fig. 4B). Briefly, slides were incubated successively for 2.5 min in methanol, 3 min in 100 % May-Gruenwald solution (Merck, Darmstadt), 3 min in 50 % May-Gruenwald solution and 15 min in 7 % Giemsa solution (Merck, Darmstadt). Slides were washed with water and mounted with CV Mount (Leica).

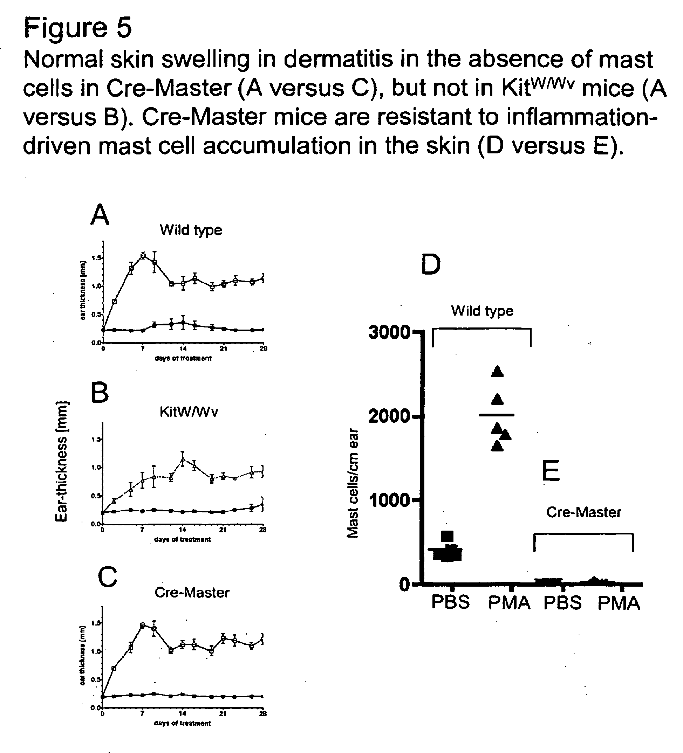

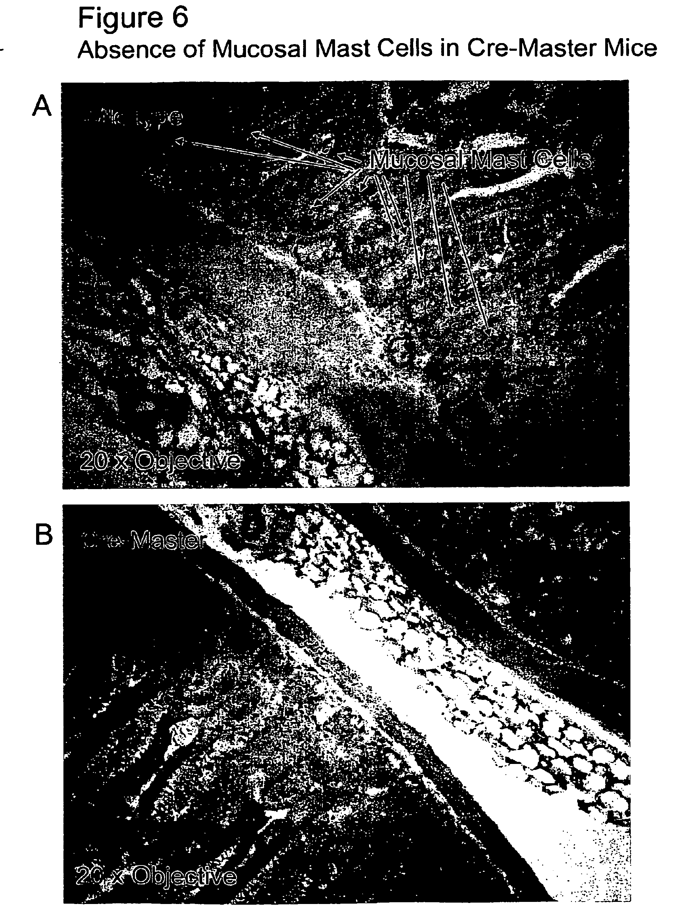

Heterozygous (Mc-cpa^{Cre/+}) and homozygous (Mc-cpa^{Cre/Cre}) Cre-Master mice lack detectable mast cells in all tissue analyzed up to now (peritoneal cavity [Figure 4], skin [Figure 5], gut mucosa [Figure 6]). The absence of mast cells has been demonstrated by histology and by flow cytometry using the mast cell markers Kit and the IgE-receptor (Fc_{ε}RI) (Figure 4A). In the peritoneal cavity fluid of wild type mice, Kit⁺Fc_{ε}RI⁺ mast cells were readily detectable (see the gate R2 in top right panel in Figure 4A). Side-by-side comparison of four Cre-Master mice revealed the complete absence of mast cells (see the gates R2 in panels Cre-Master 1-4 in Figure 4A). Peritoneal mast cells can also be visualized among cells that are centrifuged onto glass slides, and stained by May-Gruenwald-Giemsa (Figure 4B). Mast cells are typically recognized by their metachromatic (=blue dye yields purple staining) appearance. Also by this technique, mast cells were absent from the peritoneal cavity (Figure 4B).

### Absence of a cutaneous anaphylaxis reaction in Cre-Master mice

### Absence of a cutaneous anaphylaxis reaction in Cre-Master mice

Mice were sensitized at the left ear by intradermal injection of 20 ng anti-DNP IgE (SPE-7, Sigma) in 15 µl PBS. For control, pure PBS was injected into the right ear. Eighteen hours later, mice received intravenously 100 µg DNP-HSA (Sigma) and 1% Evan's blue (Sigma) in PBS. Ear photographs were taken 15 min after this antigenic challenge.

Skin mast cells can mediate anaphylactic (very fast allergic) reactions. The capacity to mount a local anaphylactic response can be measured by the passive cutaneous anaphylaxis (PCA) reaction. In this test, mice are injected intradermally into the left and right ears with immunoglobulin E (IgE) that is specific for a small antigen termed a hapten (dinitrophenyl [DNP]), or with saline (PBS) as negative control, respectively. On the next day, mice are challenged by intravenous injection of hapten-carrier (DNP-human serum albumin [HSA]) together with Evan's blue. The PCA reaction is evident by extravasation of the dye in IgE-injected but not in control PBS-injected ears. As expected, the reaction was very weak in WBB6-Kit^{W/Wv} but robust in wild type mice that mounted a strong and IgE-specific reaction. Cre-Master mice were completely unresponsive in this assay as shown by the white skin that was indistinguishable in the PBS and the IgE injected ears. Hence, in keeping with the absence of mast cells, Cre-Master mice are unable to mount this hallmark allergic reaction in the skin. Likewise, Cre-Master mice do not mount a systemic anaphylactic response which demonstrates absence of mast cells in all tissues.

### Absence of mast cells in inflamed skin of Cre-Master mice

### Absence of mast cells in inflamed skin of Cre-Master mice

One ear of each animal was treated three times per week over a period of four weeks with 10 µg phorbol 12-myristate 13-acetate (PMA, Sigma) dissolved in acetone. For controls, the contralateral ears were treated with acetone alone. Before each PMA treatment, ear thickness was determined using a caliper (Käfer Messuhrenfabrik, Villingen Schwenningen, Germany) (Fig. 5A-C). After four weeks, mice were sacrificed, and ear skin was processed for histology (Fig. 5D, E).
Ears were fixed overnight with Carnoy's fixative (ethanol: chloroform: acetic acid = 6:3:1) followed by incubation in 100 % ethanol for 8-48 h. Subsequently, specimens were embedded in paraffin. Five-micrometer paraffin sections were dewaxed by heating the glass slides to 80 °C and remaining paraffin was washed out successively with xylol and ethanol. Slides were then subjected to a descending ethanol series (96 %, 80 %, 70 %, 50 %, water) and subsequently stained for 10 min in 0.3 % aqueous toluidine solution. After brief wash with water, specimens were dehydrated with 96 % and 99 % ethanol and following a final incubation in xylol, slides were mounted with CV Mount (Leica).

In the healthy skin, mast cells are constitutively, that is, without further stimulation, present. No mast cells were found by histology in the skin of Cre-Master mice (Figure 5E ['PBS']). As noted earlier, when an irritative dermatitis is produced in normal mice by repeated topical treatment with phorbol 12-myristate 13-acetate (PMA), increased numbers of mast cells can be found at the site of inflammation (Figure 5D [compare 'PBS' and PMA']). A relatively larger increase in mast cell numbers can even be induced in the common mast cell-deficiency model, the WBB6-Kit^{W/Wv} mouse. Such inducibility of mast cell populations obviously limits the use of the available mast cell-deficiency models, and prevents their usefulness under pathological conditions that take longer than about 10 days.
To analyze whether mast cells could be induced by skin inflammation in Cre-Master mice, their skin was treated by PMA as described. While PMA-treatment induced a strong skin swelling that was in fact very similar to the wild type skin (Figure 5A versus C), no mast cells were detectable at the end of the measurement (4 weeks) in Cre-Master mice (Figure 5E). Of note, the initial skin swelling was somewhat blunted in WBB6-Kit^{W/Wv} (Figure 5B) compared to wild type (Figure 5A), and Cre-Master (Figure 5C) mice suggesting first mast cell-independent differences between the common mast cell-deficiency model (WBB6-Kit^{W/Wv}) and normal mice including Cre-Master.

### Absence of worm parasite-induced mast cells in Cre-Master mice

In contrast to the connective tissue mast cells in the skin or the peritoneal cavity, mucosal mast cells in the gut mucosa are very rare until induced by a pathological condition such as a worm infection. To examine the mucosal mast cell compartment in Cre-Master mice, mice were infected with the parasite Nippostrongylus brasiliensis. This infection lead to the appearance of mast cells in the gut mucosa in wild type mice (Figure 6A). In contrast, no mucosal mast cells were detectable in Cre-Master mice (Figure 6B). Hence, mucosal mast cells are undetectable in Cre-Master mice following Nippostrongylus brasiliensis infection.

### Examples of applications of the invention

### Cecal ligation and puncture (CLP), a model of polymicrobial septic peritonitis

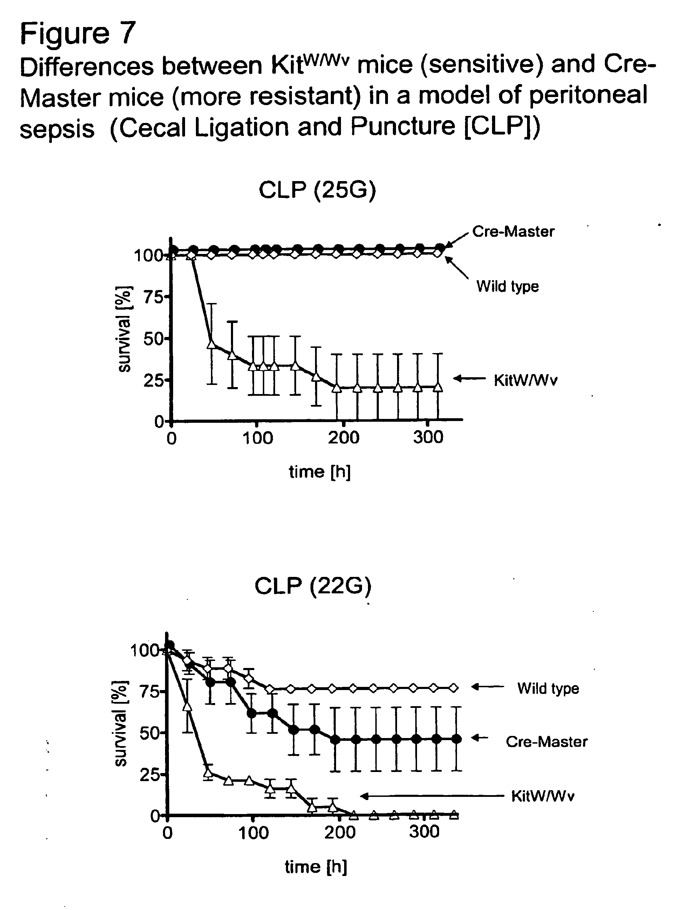

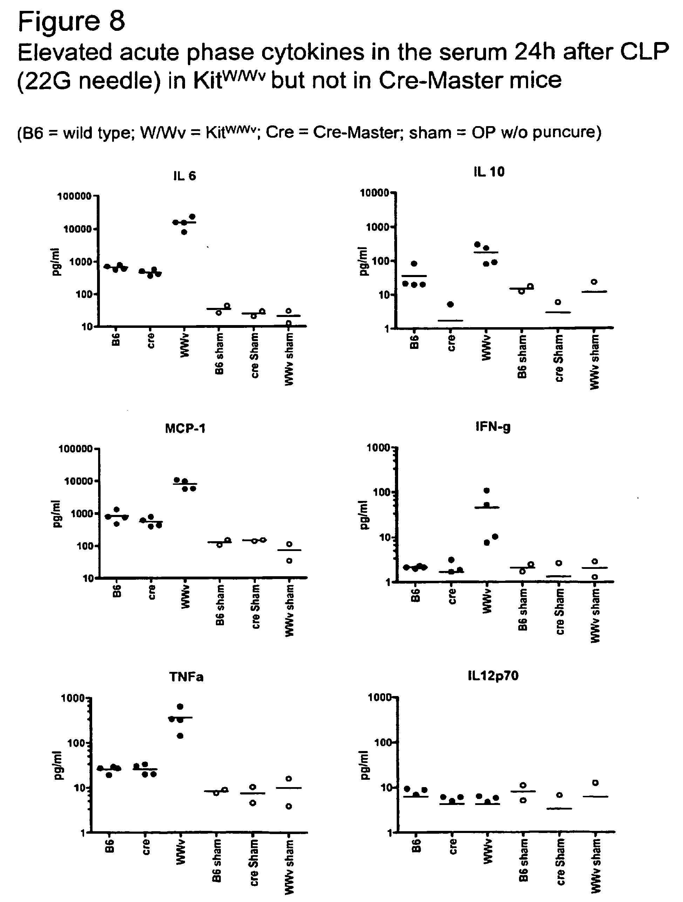

Cecal ligation and puncture was done essentially as described. Briefly, 11- to 13-week-old mice were anesthetized by i.p. injection of 100 mg/kg Ketamin (Ketanest 10 %, WDT, Garbsen, Germany) and 16 mg/kg Xylazin (Rompun 2 %, Alvetra GmbH, Neumünster, Germany) in 100 µl sterile, pyrogen-free saline. A 1-cm midline incision was made through the linea alba. The cecum was exteriorized and feces were squeezed into its posterior end. Below the ileocecal valve 1.5 cm of the distal end of the cecum were ligated with a steril 4-0 suture and punctured once with a 22 or 25 gauge needle. A small amount of stool was extruded to ensure wound patency. Sham-treated mice also had surgery done along with cecal manipulations but without ligation and puncture. The cecum was returned to the peritoneal cavity and the incision was closed with wound clips (Mikron Autoclip, Becton Dickinson). After surgery, mice received a subcutaneous injection of 1 ml saline. Mice were observed for at least 2 weeks (Fig. 7).
Acute phase cytokines were determined in the serum of mice 24 h after CLP (22 gauge). Serum was prepared using serum separator tubes (Microtainer, Bection Dickinson). The cytokines IL-6, IL-10, IL-12p70, MCP-1, IFNγ and TNFα were measured simultaneously with a cytometric bead array (CBA mouse inflammation kit, BD Biosciences) on a FACSCanto II (Becton Dickinson). Experimental procedures were according to the manufacturer's protocol. Data were analyzed with the FCAP Array Software from BD Biosciences (Fig. 8).

The concept that mast cells play important immunological roles is based, among other findings, on a model of bacterial peritoneal sepsis. In this model, the cecal part of the large intestine is partly ligated, i.e. closed, by a suture, and the wall of the ligated cecum is punctured by an injection needle of defined diameter. In this cecal ligation and puncture (CLP) model, mice of the mast cell-deficient WBB6-Kit^{W/Wv} mutant strain rapidly succumb to the developing sepsis, while most normal mice survive. It was possible to reproduce the reported survival difference between C57BL/6 (wild type) and WBB6-Kit^{W/Wv} mice (Figure 7). To test the role of mast cells in this system, mast cell-deficient mice (Cre-Master on C57BL/6) were included in the experiments. Interestingly, under mild conditions, i.e. small needle size (25G needle), only WBB6-Kit^{W/Wv} mice succumbed to sepsis, and there was no difference in the resistance to sepsis comparing wild type C57BL/6 and Cre-Master on C57BL/6 (Figure 7, top panel). Under more severe conditions (22G needle), WBB6-Kit^{W/Wv} mice died even more rapidly than under 25G-conditions (Figure 7, lower panel). Now, about 25% of the wild type C57BL/6 mice and about 50% of the Cre-Master mice died. This indicates that, while mast cells seem to play some, albeit minor role in protection from sepsis in this model, the major cause of death is Kit-related. These experiments were repeated using Cre-Master mice on the WBB6 (F1) background to exclude the possibility that WBB6 mice are more sensitive than C57BL/6 mice. The results are very clear in that WBB6 Kit^{+/+} mice (mice having normal mast cells and normal Kit expression) and WBB6 Cre-Master mice (mice lacking mast cells but with normal Kit expression) are overall protected while WBB6-Kit^{W/Wv} mice (mice lacking mast cells and lacking Kit functions) are susceptible to CLP.
The mast cell-independent susceptibility of WBB6-Kit^{W/Wv} mice to CLP-induced sepsis is further substantiated by analysis of a set of acute phase proteins that can be released in response to trauma. Interleukin-6 (IL6), IL10, MCP-1, interferon y, TNFα, and IL12p70 were measured in the peritoneal cavity (not shown), and in the serum (Figure 8). With the exception of IL12p70, all of these factors were massively induced in WBB6-Kit^{W/Wv} but only weakly induced in C57BL/6 mice, and, importantly similarly weakly in Cre-Master C57BL/6 mice. Hence, the detrimental response of WBB6-Kit^{W/Wv} mice to sepsis is unrelated to the presence or absence of mast cells. These findings provide a striking example of an application of the invention, and call for a systematic re-evaluation of the role of mast cells in many physiological and pathological processes. It is likely that Cre-Master mice can be useful for this and other purposes.

Therefore, the Cre-Master mouse is useful in order to examine
- the role of mast cells or their products in any physiological or pathological context;
- the ability of mast cells to process or modify a certain natural substrate or exogenously administered substrate or compound.
- the relevance of any of the products produced by mast cells or derived from mast cell-mediated modification of a natural substrate or said modification of an exogenously administered substrate or compound in any physiological or pathological context.
- the influence of mast cells on the progression or outcome of any of the following diseases or combinations thereof:
   Infections (e.g. bacterial, viral or fungal infections, sepsis...), inflammatory conditions or autoimmunity (e.g arthritis, inflammatory joint diseases, multiple sclerosis, diabetes, lupus erythematosus, graft rejection...), tumor immunology (growth and metastasis, rejection, invasion, angiogenesis...), vascular diseases, wound healing, tissue remodelling, aging.

## Claims

1. A non-human model animal showing a lack of mast cells, whose genome comprises, inserted into an endrogenous Mc-cPA allele ('Knocked-in'), a Cre-recombinase expression cassette comprising a Cre-recombinase coding sequence, a polyadenylation signal and optionally an intronic sequence or a marker for positive selection, whereby said Mc-cPA allele drives tissue-specific expression of said cre-recombinase coding sequence and whereby said expression eliminates the mast cells in the absence of exogenous 1oxP sites .

2. A non-human model animal according to claim 1, comprising a Cre-recombinase expression cassette, wherein the Cre-recombinase coding sequence is a codon-improved Cre-recombinase sequence.

3. A non-human model animal according to any one of claims 1 or 2, comprising a Cre-recombinase expression cassette, wherein the intronic sequence and/or the polyadenylation signal are from rabbit β-globine.

4. A non-human model animal according to any one of claims 1 to 3, comprising a Cre-recombinase expression cassette, wherein the selection marker is a neomycin resistance gene.

5. A non-human model animal according to any one of claims 1 to 4, comprising a Cre-recombinase expression cassette, wherein the selection marker is flanked by FRT-sites for subsequent removal of the selection marker by Flp-recombinase expression.

6. A non-human model animal according to any one of claims 1 to 5, comprising a recombinant Mc-cpa allele, wherein the Cre-recombinase expression cassette is inserted into the first exon of the endogenous Mc-cpa allele.

7. A non-human model animal according to any one of claims 1 to 6, comprising a recombinant Mc-cpa allele, wherein the Cre-recombinase expression cassette replaces the nucleotides +12 to +39 of the endogenous Mc-cpa allele.

8. A non-human model animal according to any one of claims 1 to 7, comprising a recombinant Mc-cpa allele, wherein the marker for positive selection has been deleted by Flp-recombinase expression.

9. A non-human model animal according to any one of claims 1 to 8, wherein the non-human animal is a rodent.

10. A non-human model animal according to any one of claims 1 to 9, wherein the non-human animal is a mouse.

11. A method for producing a non-human animal according to any one of claims 1 to 10, said method comprises the steps of
- transfection of a targeting construct comprising a Cre-recombinase expression cassette, two regions called 'arms' for homologous recombination of the targeting construct with an endogenous Mc-cPa allele specifically expressed in mast cells, a marker for positive selection and optionally a selection marker for negative selection into embryonic stem cells of a non-human animal;
- integration of the Cre-recombinase expression cassette of the targeting construct into an endogenous Mc-cpa allele by homologous recombination;
- optionally removal of the positive selection marker by expression of Flp-recombinase;
- generation of a chimeric embryo transgenic for the recombinant Mc-cpa allele;
- transfer of the said transgenic embryo into foster mothers and delivery of the chimeric animals;
- selection of the offspring of the chimeric animals for presence of the said CRE-Recombinase expression cassette inserted into the said endogenous Mc-cpa allele.

12. A method according to claim 11, wherein the embryonic stem cells are of murine origin.

13. A method according to any one of claims 11 or 12, wherein the embryonic stem cells are 12901a/Hsd E14.1 cells.

14. A method according to any one of claims 11 to 13, wherein Flp-recombinase is expressed in targeted embryonic stem cells by transient transfection of a Flp-recombinase expression vector.

15. A method according to claim 14 comprising the following steps
- transfection of a bicistronic Flp-recombinase expression vector, which comprises a promotor sequence, the Flp-recombinase sequence, an internal ribosomal entry site (IRES) and the sequence of a fluorescent protein;
- enrichment of cells transfected with the said Flp-recombinase expression vector by fluorescence activated cell sorting (FACS) for expression of the fluorescent protein.

16. A method according to claim 15, wherein the said fluorescent protein is any variant of green fluorescent protein.

17. A method according to any one of claims 11 to 16, wherein a targeting construct is used, wherein the sequences of the 'arms' are homologous to the Mc-cpa gene.

18. A method according to any one of claims 11 to 17, wherein a targeting construct is used, wherein the homologous arms are a 5' short arm and a 3' long arm.

19. A method according to any one of claims 11 to 18, wherein a targeting construct is used, wherein the short arm sequence is homologous to the nucleotides -808 to +11 of the Mc-cpa gene.

20. A method according to any one of claims 11 to 19, wherein a targeting construct is used, wherein the long arm sequence is homologous to the nucleotides +40 to +8034 of the Mc-cpa gene.

21. A method according to any one of claims 11 to 20, wherein a targeting construct is used, wherein the negative selection marker is a thymidine kinase gene.

22. Use of a non-human model animal according to any one of claims 1 to 10 for the identification of mast cells as effector-, regulator-, or target cells in a physiological or pathological context.

23. Use of a non-human model animal according to any one of claims 1 to 10 for testing the influence of mast cells on exogenously administered compounds in a physiological or pathological context.

24. Use according to any one of claims 22 or 23, wherein the influence of mast cells on the progression of an illness selected from the group consisting of bacterial infection, viral infection, fungal infection, parasitic infection, sepsis, inflammatory conditions, inflammatory joint diseases, multiple sclerosis, diabetes, lupus erythematosus, graft rejection, tumor immunology, vascular diseases, wound healing, tissue remodeling, aging and combinations thereof is tested.

## Patentansprüche

1. Ein nicht-humanes mastzellloses Tiermodell, dessen Genom, eingefügt in ein endogenes Mc-cpa-Allel ("Knock-in"), eine Cre-Rekombinase-Kassette umfassend eine Cre-Rekombinase-kodierende Sequenz, ein Polyadenylierungssignal und optional einen Intron oder einen Marker für positive Selektion, umfasst, wobei besagtes Mc-cpa-Allel für die gewebsspezifische Expression der besagten Cre-Rekombinase-kodierenden Sequenz sorgt und wobei besagte Expression die Mastzellen auch in Abwesenheit exogener loxP-Stellen eliminiert.

2. Ein nicht-humanes Tiermodell gemäß Anspruch 1, umfassend eine Cre-Rekombinase-Kassette, worin die Cre-Rekombinase-kodierende Sequenz eine Codon-optimierte Cre-Rekombinase-Sequenz ist.

3. Ein nicht-humanes Tiermodell gemäß einem der Ansprüche 1 oder 2, umfassend eine Cre-Rekombinase-Kassette, worin das Intron und/oder das Polyadenylierungssignal aus dem β-Globin-Gen des Kaninchens stammen.

4. Ein nicht-humanes Tiermodell gemäß einem der Ansprüche 1 bis 3, umfassend eine Cre-Rekombinase-Kassette, worin der Selektionsmarker ein Neomyzin-Resistenz-Gen ist.

5. Ein nicht-humanes Tiermodell gemäß einem der Ansprüche 1 bis 4, umfassend eine Cre-Rekombinase-Kassette, worin der Selektionsmarker von FRT-Stellen umgeben ist, um eine Entfernung des Selektionsmarkers durch Expression einer Flp-Rekombinase zu ermöglichen.

6. Ein nicht-humanes Tiermodell gemäß einem der Ansprüche 1 bis 5, umfassend ein rekombinantes Mc-cpa-Allel, worin die Cre-Rekombinase-Kassette ins erste Exon des endogenen Mc-cpa-Allels eingefügt ist.

7. Ein nicht-humanes Tiermodell gemäß einem der Ansprüche 1 bis 6, umfassend ein rekombinantes Mc-cpa-Allel, worin die Cre-Rekombinase-Kassette die Nukleotide +12 bis +39 des endogenen Mc-cpa-Allels ersetzt.

8. Ein nicht-humanes Tiermodell gemäß einem der Ansprüche 1 bis 7, umfassend ein rekombinantes Mc-cpa-Allel, worin der Marker für die positive Selektion durch Flp-Rekombinase-Expression entfernt wurde.

9. Ein nicht-humanes Tiermodell gemäß einem der Ansprüche 1 bis 8, wobei das nicht-humane Tier ein Nagetier ist.

10. Ein nicht-humanes Tiermodell gemäß einem der Ansprüche 1 bis 9, wobei das nicht-humane Tier eine Maus ist.

11. Verfahren zur Herstellung eines nicht-humanen Tieres gemäß einem der Ansprüche 1 bis 10, umfassend die folgenden Schritte:
- Transfektion eines Targeting-Konstrukts, umfassend eine Cre-Rekombinase-Expressionskassette, zwei homologe Regionen, genannt "Arme" für die homologe Rekombination des Targeting-Konstrukts mit einem endogenen Mc-cpa-Allel, das spezifisch in Mastzellen exprimiert wird, einem Marker für positive Selektion und optional einem Marker für negative Selektion, in embryonalen Stammzellen eines nicht-humanen Tiers;
- Integration der Cre-Rekombinase-Expressionskassette des Targeting-Konstrukts in ein endogenes Mc-cpa-Allel durch homologe Rekombination;
- optionale Entfernung des positiven Selektionsmarkers durch Expression einer Flp-Rekombinase;
- Generierung chimärer Embryonen, die transgen für das rekombinante Mc-cpa-Allel sind;
- Transferierung besagter transgener Embryonen in Ammen und Austragenlassen der chimären Tiere;
- Auswahl der Nachkommen der chimären Tiere für das besagte Mc-cpa-Allel mit besagter Cre-Rekombinase-Expressionskassette, inseriert in den besagten endogenen Mc-cpa-Lokus.

12. Verfahren gemäß Anspruch 11, wobei die embryonalen Stammzellen von der Maus stammen.

13. Verfahren gemäß einem der Ansprüche 11 oder 12, wobei die embryonalen Stammzellen 12901a/Hsd E14.1 Zellen sind.

14. Verfahren gemäß einem der Ansprüche 11 oder 13, wobei die Flp-Rekombinase durch transiente Transfektion eines Flp-Rekombinase-Expressionsvektors exprimiert wird.

15. Verfahren gemäß Anspruch 14, wobei das Verfahren folgende Schritte umfasst:
- Transfektion eines bicistronischen Flp-Rekombinase-Expressionsvektors, der eine Promotorsequenz, die Flp-Rekombinase-Sequenz, eine interne ribosomale Eintrittsstelle (engl. internal ribosomal entry site = IRES) und ein fluoreszierendes Protein enthält.
- Anreicherung der mit besagtem Flp-Rekombinase-Expressionsvektor transfizierten Zellen mittels Durchflusszytometrie (engl. fluorescenceactiviated cell sorting = FACS) für die Expression besagten fluoreszierenden Proteins.

16. Verfahren gemäß Anspruch 15, worin besagtes fluoreszierendes Protein irgendeine Variante des grün fluoreszierenden Proteins (GFP) ist.

17. Verfahren gemäß einem der Ansprüche 11 bis 16, wobei ein Targeting-Konstrukt benutzt wird, worin die Sequenz der "Arme" homolog zum Mc-cpa-Gen ist.

18. Verfahren gemäß einem der Ansprüche 11 bis 17, wobei ein Targeting-Konstrukt benutzt wird, worin die homologen "Arme" ein 5' kurzer Arm und ein 3' langer Arm sind.

19. Verfahren gemäß einem der Ansprüche 11 bis 18, wobei ein Targeting-Konstrukt benutzt wird, worin der kurze Arm homolog zu den Nukleotiden -808 bis +11 des Mc-cpa-Gens ist.

20. Verfahren gemäß einem der Ansprüche 11 bis 19, wobei ein Targeting-Konstrukt benutzt wird, worin der lange Arm homolog zu den Nukleotiden +40 bis +8034 des Mc-cpa-Gens ist.

21. Verfahren gemäß einem der Ansprüche 11 bis 20, wobei ein Targeting-Konstrukt benutzt wird, worin der negative Selektionsmarker ein Thymidinkinasegen ist.

22. Verwendung eines nicht-humanen Tiermodells gemäß einem der Ansprüche 1 bis 10 zur Identifikation von Mastzellen als Effektor-, Regulatur- oder Zielzellen in einem physiologischen oder pathologischen Kontext.

23. Verwendung eines nicht-humanen Tiermodells gemäß einem der Ansprüche 1 bis 10 zur Untersuchung des Einflusses von Mastzellen auf exogen verabreichte Substanzen in einem physiologischen oder pathologischen Kontext.

24. Verwendung gemäß einem der Ansprüche 22 oder 23, wobei der Einfluss von Mastzellen auf den Verlauf einer Krankheit aus einem der folgenden Gebiete untersucht wird: bakterielle Infektion, virale Infektion, Pilzinfektion, Parasiten-Infektion, Sepsis, entzündliche Prozesse, entzündliche Gelenkserkrankung, Multiple Sklerose, Diabetes, Lupus Erythematodes, Transplantatabstoßung, Tumorimmunologie, Gefäßerkrankung, Wundheilung, Gewebeumbildung, Alterung oder Kombinationen davon.

## Revendications

1. Un modèle animal non-humain dépourvu de mastocytes dans le génome duquel une cassette d'expression de la Cre recombinase a été insérée par « knock-in » dans le locus du gèneMc-cpa ; cette cassette conprenant la séquence codante dela Cre recombinase, un signal de polyadénylation et - optionellement - un intron ou un marqueur de sélection positive, et dont le-dit allèle Mc-cpa recombinant contrôle l'expression tissu-spécifique de la dite séquence encodant la Cre recombinase, la dite expression eliminant les mastocytes même en l'absence de sites 1oxP exogènes.

2. Un modèle animal non-humain en rapport avec la revendication 1, comprenant une cassette d'expression de la Cre recombinase, dont la séquence codant pour la Cre recombinase a été améliorée pour une expression efficace chez les eukaryotes..

3. Un modèle animal non-humain en rapport avec la revendication 1 ou 2, comprenant une cassette d'expression la Cre recombinase, où l'intron et/ou le signal de polyadénylation est/sont dérivé(s) du gène de la bêta-globine de lapin.

4. Un modèle animal non-humain en rapport avec n'importe quelle des revendications 1 à 3, comprenant une cassette d'expression la Cre recombinase, dont le marqueur de sélection est un gène de résistance à la néomycine.

5. Un modèle animal non-humain en rapport avec n'importe quelle des revendications 1 à 4, comprenant une cassette d'expression la Cre recombinase, dont le marqueur de sélection est flanqué de sites FRT pour permettre l'élimination subséquente du marqueur de sélection par l'expression de la recombinase Flp.

6. Un modèle animal non-humain en rapport avec n'importe quelle des revendications 1 à 5, comprenant un allèle recombinant Mc-cpa où la cassette d'expression la Cre recombinaseest insérée dans le premier exon de l'allèle Mc-cpa endogène.

7. Un modèle animal non-humain en rapport avec n'importe quelle des revendications 1 à 6, comprenant un allèle Mc-cpa recombinant où la cassette d'expression la Cre recombinase remplace les nucléotides +12 à +39 de l'allèle Mc-cpa endogène.

8. Un modèle animal non-humain en rapport avec n'importe quelle des revendications 1 à 7 comprenant un allèle Mc-cpa recombinant dont le marqueur de sélection positive a été délété suite à l'expression de laFlp recombinase.

9. Un modèle animal non-humain en rapport avec n'importe quelle des revendications 1 à 8 où l'animal est un rongeur.

10. Un modèle animal non-humain en rapport avec n'importe quelle des revendications 1 à 9 où l'animal est une souris.

11. Une méthode pour construire un animal non-humain en rapport avec n'importe quelle des revendications 1 à 10, ladite méthode comprenant les étapes suivantes:
◆ transfection d'une construction de ciblage comprenant une cassette d'expression la Cre recombinase, deux régions appelées "bras d'homologie" pour la recombinaison homologue de la construction de ciblage avec un allèle Mc-cpa endogène, qui est exprimé spécifiquement dans des mastocytes; un marqueur desélection positive et optionellement un marqueur de sélection négative, dans des cellules souches embryonnaires d'un animal non-humain.
◆ l'intégration de la cassette d'expression la Cre recombinasede la construction de ciblage dans un allèle Mc-cpa endogène par recombinaison homologue.
◆ l'enlèvement facultatif du marqueur de sélection positive suite à l'expression de la Flp recombinase.
◆ la création d'embryons chimères transgéniques pour l'allèle Mc-cpa recombiné.
◆ le transfert des-dits embryons transgéniques dans des souris porteuses et la naissance/mise bas des animaux chimériques.
◆ la sélection des descendants des animaux chimériques par la présencede ladite cassette d'expression de la Cre recombinaseinsérée dans ledit site Mc-cpa endogène.

12. Une méthode en rapport avec larevendication 11 où les cellules souches embryonnaires proviennent de la souris.

13. Une méthode en rapport avec les revendications 11 ou 12 où les cellules souches embryonnaires sont des cellules 129Ola/Hsd E14.1.

14. Une méthode en rapport avec les 11 ou 14 où la Flp recombinase est exprimée par transfection transitoire d'un vecteur d'expresssion de la Flp recombinase.

15. Une méthode en rapport avec la revendication 14 où ladite méthode comprenant les étapes suivantes:
◆ la transfection d'un vecteur d'expression bicistronique de la Flp recombinase contenant une séquence promotrice, la séquence codante de la Flp recombinase, un site d'entrée interne aux ribosomes (internal ribosomal entry site, IRES) et une séquence d'une protéine fluorescente.
◆ l'enrichissement desdites cellules transféctées par ledit vecteur d'expression de la Flp recombinase par tri cellulaire en cytométrie de flux (fluorescence-activated cell sorter, FACS) sur l'expression de la protéine fluorescente.

16. Une méthode en rapport avec la revendication 15 où ladite protéine fluorescente est n'importe quel variant de la protéine fluorescente verte.

17. Une méthode en rapport avec n'importe quelle des revendications 11 à 16 où une construction de ciblage est utilisée et dont les séquences des "bras" d'homologie sont homologues au gène Mc-cpa.

18. Une méthode en rapport avec n'importe quelle des revendications 11 à 17 où une construction de ciblage est utilisée et dont les bras d'homologie contienent un bras court en 5' et un bras long en 3'.

19. Une méthode en rapport avec n'importe quelle des revendications 11 à 18 où une construction de ciblage est utilisée et dont la séquence du bras court d'homologie est homologue aux nucléotides -808 /+11 du gène Mc-cpa.

20. Une méthode en rapport avec n'importe quelle des revendications 11 à 19 où une construction de ciblage est utilisée et dont la séquence du bras long d'homologie est homologue aux nucléotides +40 à +8034 du gène Mc-cpa.

21. Une méthode en rapport avec n'importe quelle des revendications 11 à 20 où une construction de ciblage est utilisée et dont le marqueur de sélection négative est le gène de la thymidine kinase.

22. L'utilisation d'un modèle animal non-humain en rapport avec n'importe quelle des revendications 1 à 10 pour identifier les mastocytes comme cellules effectrices, régulatrices ou cibles dans un contexte physiologique ou pathologique.

23. L'utilisation d'un modèle animal non-humain en rapport avec n'importe quelle des revendications 1 à 10 pour tester l'influence des mastocytes sur des composés/molécules exogènes administrés dans un contexte physiologique ou pathologique.

24. L'utilisation d'un modèle en rapport avec n'importe quelle des revendications 22 ou 23 où l'influence des mastocytes sur la progression d'une maladie est examinée parmi le groupe de pathologies suivant :: infection bactérienne, infection virale, infection fongique, infection parasitaire, septicémie, états inflammatoires, maladies articulaires inflammatoires, sclérose en plaques, diabète, lupus érythémateux, rejet aigu de greffe, immunologie des tumeurs, maladies cardiovasculaires, cicatrisation des blessures, remodelage tissulaire, vieillissement, et toute combinaison desdites maladies.
